# EUROPEAN PATENT APPLICATION

(11) **EP 4 144 379 A1**
(43) Date of publication of application: **08.03.2023**
(21) Application number: 21831927.5
(22) Date of filing: 30.03.2021
(51) Int. Cl.: A61K 49/22, A61K 49/00, A61K 41/00, A61K 9/06, B82Y 40/00, B82Y 20/00, B82Y 5/00

(54) **CONTRAST AGENT FILM-FORMING AGENT COMPOSITION, CONTRAST AGENT FILM-FORMING LIPID SOLUTION, AND CONTRAST AGENT AND PREPARATION METHOD THEREFOR**

(30) Priority: 29.06.2020 CN 202010604201
(71) Applicant: Nanjing Transcend Vivoscope Bio-Technology Co., Ltd, Nanjing, Jiangsu 211800 (CN)
(72) Inventor: DONG, Feihong, Nanjing, Jiangsu 211800 (CN); AN, Jian, Nanjing, Jiangsu 211800 (CN); ZHANG, Jiabin, Nanjing, Jiangsu 211800 (CN); GUO, Wenyu, Nanjing, Jiangsu 211800 (CN); ZHANG, Jue, Nanjing, Jiangsu 211800 (CN)
(74) Representative: Murgitroyd & Company
(86) International application number: PCT/CN2021/084087
(87) International publication number: WO 2022/001255

(57) **Abstract**

The invention relates to the field of biological medicines, and particularly relates to a film-forming agent composition for contrast agent, a film-forming lipid solution including the film-forming agent composition, a contrast agent including the film-forming lipid solution, and a preparation method thereof. The film-forming agent composition for contrast agent includes a lipid, an emulsifier and a surface charge modifier; relative to 100 parts by weight of the lipid, the content of the emulsifier is 20-50 parts by weight, and the content of the surface charge modifier is 10-35 parts by weight; and the lipid is a carboxylated phospholipid, and the surface charge modifier is a polyelectrolyte. Based on the composition, the nanodroplets of the resulting contrast agent have more uniform particle size, higher stability and better controllability, as well as a lower threshold value for ultrasonic gasification.

## Description

### FIELD OF THE INVENTION

The invention relates to the field of biomedicine, in particular to a film-forming agent composition for contrast agent, a film-forming lipid solution for contrast agent including the film-forming agent composition for contrast agent, a contrast agent including the film-forming lipid solution for contrast agent, and the preparation method thereof.

### BACKGROUND OF THE INVENTION

Recently, in the research field of ultrasound medical imaging, nanodroplets of fluorocarbon liquid wrapped by shell membrane as a novel ultrasound contrast agent of liquid-to-gas transition have been getting attention in terms of stimulus-responsive contrast-enhanced ultrasound imaging and targeted drug delivery for therapy. Compared with traditional microbubble ultrasound contrast agents, the liquid core allows nanodroplets to have smaller particle size and higher stability, and the performance improvement brings more possibilities, especially for nanodroplets carrying drugs to enter tumor tissue, thereby providing great application prospects for realizing tissue imaging and targeted precision therapy. Nanodroplet is a droplet wrapping liquid with phospholipid, protein, polymer, etc. as the main membrane material. The conventional preparation method of nanodroplets is similar to that of microbubbles, and nanodroplets may be prepared by ultrasonic cavitation method, homogenization method, or condensation method. These nanodroplets may be used for stimulus-responsive contrast-enhanced ultrasound imaging and targeted drug delivery for therapy.

However, the current application of nanodroplets usually has problems such as broad particle size distribution, poor controllability of carrier experiments, and too high activation threshold to achieve imaging under ultrasonic response. Therefore, for the development of nanodroplets, for scientific research or clinical application, there is an urgent need for an ultrasound contrast agent of nanodroplets which may be activated by ultrasound and has a uniform particle size.

### BRIEF DESCRIPTION OF THE INVENTION

The object of the present invention is to overcome the above problems of the existing nanodroplets, and to provide a film-forming agent composition for contrast agent, a film-forming lipid solution for contrast agent including the film-forming agent composition for contrast agent, a contrast agent including the film-forming lipid solution for contrast agent, and a preparation method thereof. The composition and preparation method according to the present invention may make the resulting nanodroplets of the contrast agent have more uniform particle size, higher stability and better controllability; moreover, contrast agent according to the present invention not only has a lower vaporization threshold stimulated by ultrasound pulse to be used as an ultrasound contrast agent, but also may better meet the needs of clinical and scientific research for an ultrasound contrast agent of liquid-to-gas transition; and in a preferred embodiment, it has good optical control properties and may be used as a photoacoustic contrast agent or a multi-modal contrast agent which has a broad prospect of application.

The inventors of the present invention have found that by combining specific lipids, emulsifiers and surface charge modifiers with specific ratio, better Ouzo effect may be produced by the resulting film-forming agent composition for contrast agent in the process of preparing the contrast agent, thereby obtaining a contrast agent with better properties.

Accordingly, a first aspect of the present invention provides a film-forming agent composition for contrast agent, which includes a lipid, an emulsifier and a surface charge modifier; wherein relative to 100 parts by weight of the lipid, the content of the emulsifier is 20 to 50 parts (e.g., 20, 25, 30, 35, 40, 45, 50 parts) by weight, and the content of the surface charge modifier is 10 to 35 parts (e.g., 10, 15, 20, 25, 30, 35) by weight; and wherein the lipid is a carboxylated phospholipid, and the surface charge modifier is a polyelectrolyte.

The specific selection and ratio of the above lipid, emulsifier and surface charge modifier according to the present invention may achieve better effects. In order to further improve the comprehensive performance of the contrast agent, preferably, relative to 100 parts by weight of the lipid, the content of the emulsifier is 25 to 45 parts by weight, and the content of the surface charge modifier is 15 to 30 parts by weight; more preferably, relative to 100 parts by weight of the lipid, the content of the emulsifier is 30 to 40 parts by weight, and the content of the surface charge modifier is 22 to 28 parts by weight.

In the present invention, the lipids may be various carboxylated phospholipids. Preferably, the lipid is a carboxylated phospholipid, which is any one or more selected from the group consisting of: 1,2-distearoyl-sn-glycero-3-phosphocholine (DSPC), distearoylphosphatidylethanolamine (DSPE), dipalmitoylphosphatidylcholine (DPPC), 1,2-bis(diphenylpho sphine)ethane (DPPE), and distearoylphosphatidylethanolamine-polyethylene glycol (DSPE-PEG).

The object of the present invention can be achieved by including carboxyl groups in the lipid. Preferably, the single lipid molecule includes 1 to 3 carboxyl groups, more preferably one carboxyl group. The content of carboxyl groups in the lipid is determined by infrared spectroscopy.

In the present invention, the emulsifier may be an emulsifier commonly used in the art. Preferably, the emulsifier is any one or more selected from the group consisting of: polyethylene glycol 4000 (PEG4000), polyethylene glycol 40s (PEG40s), polyoxypropylene polyoxyethylene block polyether (Pluronic), polyethylene glycol 1400 (PEG1400), and polysorbate-80.

In the present invention, the term "polyelectrolyte" is a conventional concept in the art, i.e., a long-chain polymer with ionizable groups, which will be ionized in a polar solvent so that the polymer chain is charged.

Various polyelectrolytes providing positive or negative charges may be used in the present invention as surface charge modifiers. In a preferred embodiment, in order to better cooperate with other components, the surface charge modifier is any one or more selected from the group consisting of: hyaluronic acid, chitosan, sodium hydroxymethyl cellulose, carbomer, sodium alginate, polyamine, and hard amine; more preferably, the surface charge modifier is any one or more selected from the group consisting of: sodium alginate, polyethyleneimine, and polyallylamine hydrochloride.

The inventors of the present invention also found that, by introducing a photosensitizer into the film-forming agent composition for contrast agent, it may not only help the nanodroplets have more uniform particle size, higher stability and better controllability, but also make the nanodroplets of contrast agents have the properties of optical control. Particularly, the inventors of the present invention found that, nanodroplets including photosensitizers may undergo phase transition under the stimulation of laser light, thereby gasifying into microbubbles. If ultrasound is combined with laser stimulation, the difficulty of activating nanodroplets may be greatly reduced. Therefore, the contrast agent obtained from the specific embodiment including the photosensitizer is a multi-modal contrast agent, which has a broader application prospect.

Based on this, according to a preferred embodiment, the film-forming agent composition for contrast agent further includes a photosensitizer. Relative to 100 parts by weight of the lipid, the content of the photosensitizer is 10 to 35 parts by weight, more preferably 15 to 30 parts by weight; still more preferably 22 to 28 parts by weight.

The photosensitizer may be various photosensitizers commonly used in the art, preferably, in order to have a better synergistic effect with other components of the present invention, is any one or more selected from: methylene blue, porphyrin, and derivatives thereof. For example, the derivatives of the porphyrin include, but are not limited to: hematoporphyrin, photoporphyrin, mesoporphyrin, sodium porphyrin, gallium porphyrin, hydrophilic chlorin derivative, protoporphyrin, and copper protoporphyrin.

Further, the inventors of the present invention also found that, when the gold particles modified with amino groups on the surface are introduced into the lipid shell of a nanodroplet of the contrast agent, on one hand, the shell thickness of a nanodroplet of the multi-modal contrast agent may be made to be adjustable, and on the other hand, the multi-modal contrast agent may have better photosensitivity property.

Based on this, according to another preferred embodiment, the film-forming agent composition for contrast agent further includes gold particles modified with amino groups on the surface. Relative to the total weight of the lipid, emulsifier, surface charge modifier and photosensitizer (if any) as 100 parts by weight, the content of the gold particles is 20 to 50 parts (e.g., 20, 22, 24, 26, 28, 30, 40, 42, 44, 46, 48, 50 parts) by weight, more preferably 30 to 40 parts (e.g., 30, 31, 32, 33, 34, 35, 36, 37, 38, 39, 40 parts).

Preferably, each of the gold particles includes 1 to 20 (e.g., 1, 2, 3, 4, 5, 6, 7, 8, 9, 10, 11, 12, 13, 14, 15, 16, 17, 18, 19, 20) amino groups, more preferably 3 to 6 amino groups. The content of amino groups is determined by infrared spectroscopy.

Preferably, the particle size of the gold particles is 1nm to 30 nm, more preferably 5nm to 20 nm.

In the present invention, the term "particle size" and "average particle size" have different meanings. The term "particle size" refers to the geometric spherical diameter of a single particle rather than an average value; when it is a range value, it means that the particle sizes of the particles in the same material fall within the range; meanwhile, the present invention allows a certain amount of error, i.e., when the particle sizes of less than 5% of the total number are not within the required range, it is also considered to meet the requirements; the particle size is measured by transmission electron microscopy (TEM). The term "average particle size" refers to the average value of the particle sizes of all particles in a system, and the average particle size in the present invention refers to the number-average particle size, which is determined by particle size analyzer (Malvern, Mastersizer 3000) based on the principle of dynamic light scattering (DLS).

According to a specific embodiment, in the process of preparing the contrast agent, the gold particles modified with amino groups on the surface are added separately in the later stage of preparation; thus in the film-forming agent composition for ultrasound contrast agent, the gold particles modified with amino groups on the surface are stored independently from other components, and other components may also be stored independently of each other.

In the present invention, the photosensitizer and the gold particles do not necessarily exist at the same time.

The film-forming agent composition for contrast agent according to the present invention may further include a drug. The drug may be various drugs as required for actual treatment. For example, the drugs include, but are not limited to, paclitaxel, doxorubicin, bleomycin, and the like. All drugs used in contrast agents in the art may be used in the present invention, and those skilled in the art may select the specific substances and amounts as required.

The film-forming agent composition for contrast agent according to the present invention may also include other conventional adjuvants in the art; as long as the performance of other components is not adversely affected, those skilled in the art may choose these other adjuvants and the contents of them may refer to the conventional contents in the art.

The second aspect of the present invention provides a film-forming lipid solution for contrast agent, which includes the film-forming agent composition for contrast agent according to the first aspect of the present invention.

As long as the film-forming lipid solution for contrast agent includes the film-forming agent composition for contrast agent according to the first aspect of the present invention, it falls within the protection scope of the film-forming lipid solution for contrast agent according to the second aspect of the present invention; the film-forming lipid solution for contrast agent may also include ingredients conventionally added in the art, such as solvent, adjuvant, drug, and the like.

For the needs of production and sales, in the present invention a product may be produced and sold in the form of the film-forming agent composition for contrast agent according to the first aspect of the present invention; a product may also be produced and sold in the form of the film-forming lipid solution for contrast agent according to the second aspect of the present invention for production and sales (for example, a product obtained by step (1) in the method according to the fourth aspect of the present invention); and a product may also be produced and sold in the form of the contrast agent according to the third aspect of the present invention for production and sales.

When a product is produced and sold in the form of the film-forming lipid solution for contrast agent according to the second aspect of the present invention, it needs to be prepared into a contrast agent before use; for example, the contrast agent may be prepared by applying mechanical force in a conventional manner in the art (e.g., ultrasonic cavitation method, homogenization method, condensation method, and the likes), or it may be produced by the method according to the fourth aspect of the present invention. The advantage of a product for production and sales in the form of a film-forming lipid solution for contrast agent lies in that: it is easier to store such a product for a long time than a contrast agent.

A third aspect of the present invention provides a contrast agent including nanodroplets, wherein the nanodroplet consists of a shell and the content wrapped by the shell, and the shell is produced by the film-forming lipid solution for contrast agent according to the second aspect of the present invention.

In the present invention, the contrast agent consists of a continuous phase and a dispersed phase; the dispersed phase is the nanodroplets, and the continuous phase may be a conventional continuous phase used to prepare an ultrasound contrast agent in the field, such as phosphate (PBS) buffer solution.

In the present invention, the film-forming lipid solution for contrast agent according to the second aspect of the present invention may be combined with various conventional contents in the field to produce a contrast agent by various conventional preparation methods in the field, and they all fall within the protection of the present invention.

The content may be a biocompatible substance which includes drug or does not include drug and is a gaseous state or a phase-changeable liquid. For example, the biocompatible substance is any one or more selected from the group consisting of: air, nitrogen, carbon dioxide, oxygen, hydrogen, nitrogen oxide, inert gas, halosilane, haloalkane, and sulphur halide. The term "phase-changeable liquid biocompatible substance" refers to a substance that may be a liquid state at normal temperature and pressure or a specific environmental condition at normal temperature, but it may be converted into a gaseous state at normal temperature under a specific environment or excitation conditions, e.g., perfluorocarbon. Perfluorocarbon also has the properties of high molecular weight, stable quality, and good biological safety. The contrast agent obtained with perfluorocarbon liquid as the content has the unique property of liquid-to-gas transition, and may be excited to form a gaseous state under ultrasound, light and other conditions, thereby forming a microbubble contrast agent. The inertial cavitation occurs upon rupture of a microbubble, so that the microbubble loads drug to blast at the target area and release the drug locally, thereby exhibiting the therapeutic effect of targeted drug delivery. The cavitation of the microbubble locally generates microflow and shear force, thereby stimulating the channels between the pores of the endothelial cell membrane and cells to be opened, and facilitating the delivery of the therapeutic drug into the cell. Therefore, the contrast agent obtained with perfluorocarbon liquid as the content may not only achieve a similar effect to the traditional microbubble ultrasound contrast agent, but also its liquid core allows a nanodroplet to have a smaller particle size and higher stability, thereby providing a huge application prospect for realizing tissue imaging and targeted precision therapy; accordingly, it is specially studied in the present invention. Therefore, according to a preferred embodiment of the present invention, the content is a perfluorocarbon liquid.

Preferably, the perfluorocarbon is any one or more selected from the group consisting of: perfluoropropane, perfluorobutane, perfluoropentane, and perfluorohexane; wherein when the perfluorocarbon is perfluoropentane and/or perfluorohexane, since these two substances are in liquid state at normal temperature and pressure, there is no need for special operation in the process of preparing a film-forming agent for contrast agent and the contrast agent; however, when the perfluorocarbon is perfluoropropane and/or perfluorobutane, since these two substances are gaseous at normal temperature and pressure, it is necessary to adjust and control the temperature and/or pressure during the process of preparing a film-forming agent for contrast agent and the contrast agent so as to keep them in a liquid state.

At present, when preparing nanodroplets with fluorocarbon liquid wrapped by shell membrane as ultrasound contrast agent, the method of applying mechanical force in a conventional manner (e.g., ultrasonic cavitation method, homogenization method, condensation method, and the likes) is still used for preparing gas content, so as to achieve the emulsification of the membrane material and self-assembly to form droplets. However, the inventors of the present invention found that, the particle size distribution of the nanodroplets obtained by this rough method is very wide, thus the controllability of the carrier experiments is poor, which also greatly increases the difficulty of the application of nanodroplets; moreover, the activation threshold of the nanodroplets obtained by this method is too high, and usually the high energy activation of single-sensor ultrasonic probe (usually the corresponding mechanical index is greater than 1.9) is required to realize the liquid-to-gas transition of the perfluorocarbon in the nanodroplets, and it is difficult to achieve imaging under ultrasound response. Therefore, with intensive research the inventors of the present invention have found a method for preparing a contrast agent through the Ouzo effect, and also found a film-forming agent composition for contrast agent suitable for the method, so that the present invention may obtain an ultrasound contrast agent or multi-modal contrast agent with fluorocarbon liquid wrapped by shell membrane, which has more uniform particle size, higher stability, better controllability, and lower threshold of ultrasonic vaporization.

A fourth aspect of the present invention provides a method for preparing a contrast agent, wherein the method includes the following steps:
(1) mixing the film-forming agent composition for contrast agent according to the first aspect of the present invention with a first organic solvent to obtain a film-forming lipid solution for contrast agent;
(2) mixing the film-forming lipid solution for contrast agent with perfluorocarbon liquid to obtain solution **A;**
(3) mixing the solution **A** and the hydration solution to produce the Ouzo effect, leaving the resulting material to stand for stratification, and separating the lipid phase to obtain material **B;**
(4) resuspending the material **B** in a buffer solution to obtain an initial nanodroplet solution;
(5) contacting the initial nanodroplet solution with gold particles modified with amino groups on the surface and an initiator.

In step (1), preferably the first organic solvent is any one or more selected from the group consisting of: ethanol, isopropanol and triethanolamine.

In step (1), the amount of the organic solvent is not particularly limited, as long as the composition according to the present invention may be dissolved; preferably the amount of the first organic solvent is such that the concentration of the film-forming agent composition for contrast agent in terms of lipid is 0.5-8 (e.g., 0.5, 1, 2, 3, 4, 5, 6, 7, 8) mg/mL, preferably 1-5 mg/mL, and most preferably 2-4 (e.g., 2, 2.2, 2.4, 2.6, 2.8, 3, 3.2, 3.4, 3.6, 3.8, 4) mg/mL.

In step (2), preferably the saturability of perfluorocarbon in the solution **A** is 30-100%, more preferably 60-90% (e.g., 60%, 65%, 70%, 75%, 80%, 85%, 90%).

In the present invention, the saturability of the perfluorocarbon refers to, in a unit volume, the mass ratio of the perfluorocarbon dissolved in the solution and the perfluorocarbon in the solution when it reaches saturation.

According to a specific embodiment, the method for controlling the saturability includes: first fully mixing the film-forming lipid solution for contrast agent and excess perfluorocarbon liquid to obtain a layered liquid, discarding the excess perfluorocarbon liquid in the lower layer, and taking the upper layer liquid as perfluorocarbon saturated liquid (saturability is 100%); then, mixing Y volume of perfluorocarbon saturated liquid with Z volume of film-forming lipid solution for contrast agent to prepare perfluorocarbon solution **A** with a saturability of a%; wherein a%=Y/(Y+Z)×100%, the unit of Y is mL, and the unit of Z is mL.

In step (3), the hydration solution may be a hydration solution commonly used in the art; preferably, the hydration solution includes glycerol, propylene glycol and phosphate; more preferably the volume ratio of glycerol, propylene glycol and phosphate is 1:(0.5-3):(5-12); still more preferably 1:(1-2):(7-10).

In step (3), preferably the volume ratio of the solution **A** to the hydration solution is 1:(0.3-2)(e.g., 1:0.3, 1:0.4, 1:0.5, 1:0.6, 1:0.7, 1:0.8, 1:0.9, 1:1, 1:1.1, 1:1.2, 1:1.3, 1:1.4, 1:1.5, 1:1.6, 1:1.7, 1:1.8, 1:1.9, 1:2), more preferably 1:(0.5-1).

In step (3), the time for standing and stratification does not need to be specially limited, and the stratification may usually be completed within 10-30 min.

In step (3), preferably the method of separating the lipid phase includes: sucking out the upper layer solution obtained by layering and discarding it; centrifuging the lower layer liquid (e.g., centrifugation speed is 3000-5000rpm), sucking out the upper layer solution again and discarding it, thereby obtaining the material B.

In step (4), preferably the buffer solution is a phosphate buffer solution.

In step (4), preferably the resuspending process includes: after resuspending the material **B** in the buffer solution, centrifuging, discarding the aqueous phase solution, adding the buffer solution again to resuspend; repeating the resuspending- centrifuging steps for 2-4 times to obtain an initial nanodroplet solution.

In step (4), the amount of the buffer solution is not particularly limited, preferably the amount of the buffer solution is such that the lipid concentration in the mixture is 0.1-6 (e.g., 0.1, 0.5, 1, 2, 3, 4, 5, 6) mg/mL, preferably 0.5-4 mg/mL, most preferably 2-4 mg/mL.

In step (5), preferably the initiator is obtained by combining 1-(3-dimethylaminopropyl)-3-ethylcarbodiimide hydrochloride (EDC) and N-hydroxysuccinimide (NHS) in a weight ratio of 1:(0.5-4) (e.g., 1:0.5, 1:0.75, 1:0.8, 1:0.9, 1:1, 1:1.2, 1:1.5, 1:2, 1:2.5, 1:3, 1:4), more preferably 1:(0.75-1.5).

In step (5), the amount of the gold particles modified with amino groups on the surface is selected according to the definition in the film-forming agent composition for contrast agent in the first aspect of the present invention. Preferably, the weight ratio of the amount of the initiator to the gold particles modified with amino groups on the surface is (0.5-3):1 (e.g., 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 3:1), more preferably (1-2):1.

In step (5), preferably the contact conditions include: a temperature of 10-35°C, and a time of 21-36h. Preferably, the contacting manner is standing, i.e., the mixed materials are incubated at the temperature for said time.

Any one of the contrast agents according to the third aspect of the present invention may be prepared by the method according to the fourth aspect of the present invention.

Preferably, the average particle size of the nanodroplets of the contrast agent is 50-500 nm, more preferably 100-400 nm. The particle size is determined by a particle size analyzer based on the DLS principle.

The particle size of the nanodroplets of the contrast agent according to the present invention has strong controllability, for example, it may be adjusted by adjusting the lipid concentration and the perfluorocarbon saturation in the film-forming lipid solution for contrast agent. Particularly, in general the particle size of nanodroplets increases with increasing of lipid concentration, and firstly increases and then decreases with increasing of perfluorocarbon saturation.

Preferably, the polydispersity coefficient for the particle size of the nanodroplets of the contrast agent is 0.05-0.4, and in a preferred embodiment it may reach 0.1-0.3. In the present invention, the polydispersity coefficient refers to the degree of dispersion of the particle size of the nanodroplets, which is expressed by the Dw/Dn formula, (wherein Dw and Dn are respectively the weight-average and number-average particle diameters), which may be determined and calculated by the particle size analyzer based on the DLS principle.

Preferably, the shell thickness of the nanodroplets of the contrast agent is 5-20 nm, more preferably 8-15 nm. The shell thickness is measured by TEM. The shell thickness may be adjusted as required, e.g., by increasing the content of gold particles to increase the shell thickness.

Through the above technical solutions, compared with the prior art the present invention has at least the following advantages:
(1) the nanodroplets of the contrast agent according to the present invention have a suitable particle size range and higher uniformity;
(2) the particle size of the nanodroplets of the contrast agent according to the present invention has stronger controllability, and the particle size may be adjusted as required;
(3) the surface tension and thickness of the shell of the nanodroplets of the contrast agent according to the present invention may be adjusted by adjusting the amount of the emulsifier and the photosensitizer, so that the threshold of ultrasonic vaporization of the nanodroplets is controllable;
(4) the contrast agent according to the invention may realize imaging under the activation of the ultrasonic imaging probe, thereby obtaining obvious and clear ultrasonic contrast images;
(5) the contrast agent according to the present invention may not only be used as an ultrasound contrast agent, but also in a preferred embodiment it may be used as a photoacoustic contrast agent and a multi-modal contrast agent, and has a broader application prospect;
(6) in the present invention, the contrast agent is prepared by a method based on Ouzo effect, and the reaction conditions are mild.

The endpoints of a range and any values disclosed herein are not limited to the precise ranges or values, which are to be understood to encompass values proximate to those ranges or values. For value ranges, the endpoints of each range, an endpoint of each range and an individual point value, and the individual point values may be combined with each other to yield one or more new value ranges which should be considered as particularly disclosed herein.

### BRIEF DESCRIPTION OF DRAWINGS

Fig. 1 is an image of contrast agent I1 obtained in Example 1 under a transmission electron microscope;
Fig. 2 is an image of the contrast agent I1 obtained in Example 1 before activation (a) and after activation (b).

### DETAILED DESCRIPTION OF THE INVENTION

The present invention will be described in detail below by the Examples. The described Examples of the present invention are only a part of the examples of the present invention, but not all of the examples. Based on the Examples of the present invention, all other examples obtained by those of ordinary skill in the art without creative efforts shall fall within the protection scope of the present invention.

Unless otherwise specified, the materials used in the following Examples are all commercially available analytical grades.

The PBS buffer solution used in the following Examples is prepared by the following method: weighing 0.097g of KCl, 4.005g of NaCl, 1.145g of Na₂HPO_{4·}H₂O and 0.096g of KH₂PO₄ to a beaker of 1L, adding deionized water and making up to 500ml to prepare phosphate buffered saline (PBS solution) for use (if it is not enough, it may be prepared multiple times).

### Example 1

(1) Carboxylated dipalmitoyl phosphatidyl choline (DPPC) (lipid; 1 carboxyl group per molecule, CAS: 63-89-8, purchased from SigmaAldrich), polyethylene glycol 40s (PEG40s) (emulsifier), sodium alginate (surface charge modifier, CAS: 9005-38-3) and methylene blue (photosensitizer) are mixed in a weight ratio of 100:35:25:25 to fully dissolve in the organic solvent ethanol (the amount of organic solvent is such that the concentration of lipid is 2mg/mL), thereby obtaining a film-forming lipid solution for contrast agent;
(2) Part of the film-forming lipid solution for contrast agent is taken out, and excess perfluorohexane liquid is added to obtain a saturated perfluorocarbon solution; the saturated perfluorocarbon solution is mixed with the remaining film-forming lipid solution for contrast agent to prepare solution **A** with 80% saturability;
(3) The solution **A** is mixed with a hydration solution (a mixture of glycerol, propylene glycol and PBS buffer in a volume ratio of 1:1.5:8.5) in a volume ratio of 1:0.8, allowing to stand for 20 min; the solution is divided into two layers, sucking off the upper layer solution, and centrifuging the lower layer liquid for 10min at a speed of 4000rpm; then the upper layer solution is sucked off again to obtain material B;
(4) The material B is resuspended in PBS buffer solution, centrifuged, and the upper layer solution is sucked off, then the obtained mixture is added into PBS buffer solution again to be resuspended and centrifuged; this process is repeated for three times; the finally obtained lower layer material (referred to as material C) is mixed with PBS buffer solution to make the lipid concentration in the mixed solution to be 4 mg/mL, and to be resuspended to obtain the initial nanodroplet solution;
(5) Gold particles (whose surface modified with amino groups, each gold particle includes 3 amino groups, and the average particle size of the gold particles is about 10 nm; manufacturer: SigmaAldrich; product number: 765309) and an initiator (consisting of EDC and NHS in a weight ratio of 1:0.75) are added to the initial nanodroplet solution according to material C: gold particles: initiator in a weight ratio of 10:3:4, the obtained mixture being incubated at room temperature 20°C for 24 h to obtain a contrast agent, denoted as I1.

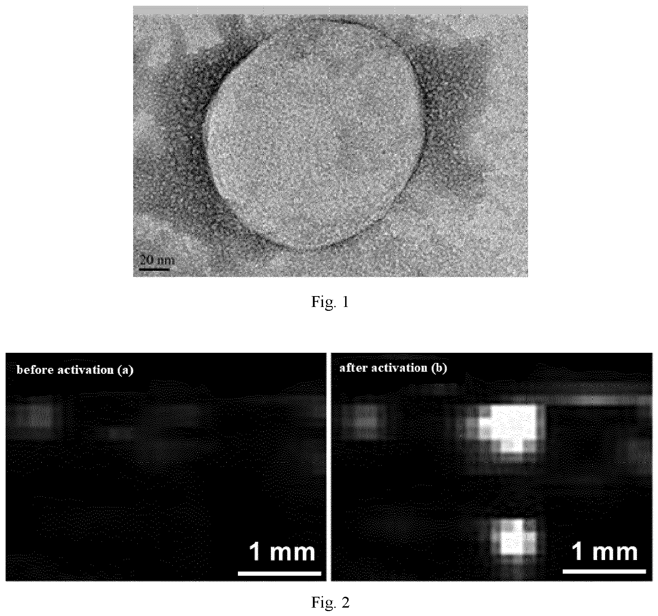

The image of the contrast agent I1 under the transmission electron microscope is shown in Fig. 1. It can be seen from Fig. 1 that the nanodroplets are spheres with a core-shell structure.

### Example 2

(1) Carboxylated 1,2-distearoyl-sn-glycero-3-phosphocholine (DSPC) (lipid; 1 carboxyl group per molecule, CAS: 816-94-4, purchased from SigmaAldrich), polyethylene glycol 4000 (PEG4000) (emulsifier), polyethyleneimine (surface charge modifier, CAS: 9002-98-6) and methylene blue (photosensitizer) are mixed in a weight ratio of 100:30:28:22 to fully dissolved in the organic solvent isopropanol (the amount of organic solvent is such that the concentration of lipid is 4 mg/mL), thereby obtaining a film-forming lipid solution for contrast agent;
(2) Part of the film-forming lipid solution for contrast agent is taken out, and excess perfluorohexane liquid is added to obtain a saturated perfluorocarbon solution; the saturated perfluorocarbon solution is mixed with the remaining film-forming lipid solution for contrast agent to prepare solution **A** with 90% saturability;
(3) The solution **A** is mixed with a hydration solution (a mixture of glycerol, propylene glycol and PBS buffer in a volume ratio of 1:1:10) in a volume ratio of 1:1, allowing to stand for 20 min; the solution is divided into two layers, sucking off the upper layer solution, and centrifuging the lower layer liquid for 10min at a speed of 4000rpm; then the upper layer solution is sucked off again to obtain material B;
(4) The material B is resuspended in PBS buffer solution, centrifuged, and the upper layer solution is sucked off, then the obtained mixture is added into PBS buffer solution again to be resuspended and centrifuged; this process is repeated for three times; the finally obtained lower layer material (referred to as material C) is mixed with PBS buffer solution to make the lipid concentration in the mixed solution to be 3 mg/mL, and to be resuspended to obtain the initial nanodroplet solution;
(5) Gold particles (whose surface modified with amino groups, each gold particle includes 3 amino groups, and the average particle size of the gold particles is about 20 nm; SigmaAldrich product number: 765341) and an initiator (consisting of EDC and NHS in a weight ratio of 1:0.85) are added to the initial nanodroplet solution according to material C: gold particles: initiator in a weight ratio of 10:4:6, the obtained mixture being incubated at room temperature 20°C for 24 h to obtain a contrast agent, denoted as 12.

### Example 3

(1) Carboxylated distearoylphosphatidylethanolamine (DSPE) (lipid; 1 carboxyl group per molecule, CAS: 1069-79-0, purchased from SigmaAldrich), Pluronic-F68 (emulsifier), polyallylamine hydrochloride (surface charge modifier, CAS: 30551-89-4) and porphyrin (photosensitizer) are mixed in a weight ratio of 100:40:22:28 to fully dissolve in the organic solvent triethanolamine (the amount of organic solvent is such that the concentration of lipid is 4mg/mL), thereby obtaining a film-forming lipid solution for contrast agent;
(2) Part of the film-forming lipid solution for contrast agent is taken out, and excess perfluorohexane liquid is added to obtain a saturated perfluorocarbon solution; the saturated perfluorocarbon solution is mixed with the remaining film-forming lipid solution for contrast agent to prepare solution **A** with 60% saturability;
(3) The solution **A** is mixed with a hydration solution (a mixture of glycerol, propylene glycol and PBS buffer in a volume ratio of 1:2:7) in a volume ratio of 1:0.5, allowing to stand for 20 min; the solution is divided into two layers, sucking off the upper layer solution, and centrifuging the lower layer liquid for 10min at a speed of 4000rpm; then the upper layer solution is sucked off again to obtain material B;
(4) The material B is resuspended in PBS buffer solution, centrifuged, and sucking off the upper layer solution is sucked off, then the obtained mixture is added into PBS buffer solution again to be resuspended and centrifuged; this process is repeated for three times; the finally obtained lower layer material (referred to as material C) is mixed with PBS buffer solution to make the lipid concentration in the mixed solution to be 3.5 mg/mL, and to be resuspended to obtain the initial nanodroplet solution;
(5) Gold particles (whose surface modified with amino groups, each gold particle includes 3 amino groups, and the average particle size of the gold particles is about 20 nm; manufacturer: SigmaAldrich; product number: 765341) and an initiator (consisting of EDC and NHS in a weight ratio of 1:1) are added to the initial nanodroplet solution according to material C: gold particles: initiator in a weight ratio of 10:3:6, the obtained mixture being incubated at room temperature 20°C for 24 h to obtain a contrast agent, denoted as I3.

### Example 4

Referring to the method of Example 1, the difference is that the weight ratio of lipid, emulsifier, surface charge modifier and photosensitizer is changed to 100:23:12:12, and the total weight is kept unchanged.

Finally, a contrast agent is obtained and denoted as I4.

### Example 5

Referring to the method of Example 1, the difference is that the weight ratio of lipid, emulsifier, surface charge modifier and photosensitizer is changed to 100:46:32:32, and the total weight is kept unchanged.

Finally, a contrast agent is obtained and denoted as I5.

### Example 6

Referring to the method of Example 1, the difference is that in step (5), the gold particles modified with amino groups on the surface are replaced with the same weight of silver particles with amino groups modified on the surface (Yoshikura Nano, JCSNP03-0010).

Finally, a contrast agent is obtained and denoted as I6.

### Example 7

Referring to the method of Example 1, the difference is that in step (2), the saturability of the solution **A** is changed to 40%.

Finally, a contrast agent is obtained and denoted as I7.

### Example 8

Referring to the method of Example 1, the difference is that in step (2), the saturability of the solution **A** is changed to 100%.

Finally, a contrast agent is obtained and denoted as I8.

### Example 9

Referring to the method of Example 1, the difference is that no photosensitizer is added.

Finally, a contrast agent is obtained and denoted as I9.

### Example 10

Referring to the method of Example 1, the difference is that the amount of the photosensitizer is changed so that the weight ratio of lipid to photosensitizer is 100:20.

Finally, a contrast agent is obtained and denoted as I10.

### Example 11

Referring to the method of Example 1, the difference is that the amount of the photosensitizer is changed so that the weight ratio of lipid to photosensitizer is 100:60.

Finally, a contrast agent is obtained and denoted as I11.

### Example 12

Referring to the method of Example 1, the difference is that no gold particles are added.

Finally, a contrast agent is obtained and denoted as I12.

### Comparative Example 1

With reference to the method of Example 1, the difference is that no surface charge modifier, emulsifier and photosensitizer are added; particularly, in step (1), the DPPC with a weight equal to the total weight of DPPC, PEG40s, sodium alginate and methylene blue in Example 1 is fully dissolved in the same amount of organic solvent ethanol as in Example 1, thereby obtaining a film-forming lipid solution for contrast agent.

Finally, a contrast agent is obtained and denoted as D1.

### Comparative Example 2

A contrast agent is prepared by applying mechanical external force, referring to a preparation method described by Geoffrey P. et al., Supporting information, Super-resolution ultrasound imaging in vivo with transient laser-activated nanodroplets, Nano letters, 2016, 16, 4, 2556-2559. Particularly, the method includes:
(D-1) According to step (1) of Example 1 and referring the above article of Geoffrey P. et al., the lipid DPPC is replaced with the same weight of water, thereby obtaining a film-forming lipid solution for contrast agent;
(D-2) In a 180W ultrasonic bath, the film-forming lipid solution for contrast agent is mixed with a perfluorohexane liquid (the perfluorohexane is excessive with reference to the above article of Geoffrey P. et al.), thereby obtaining D-A solution;
(D-3) The D-A solution is vortexed for 10 seconds, and sonicated in an ultrasonic bath for 5 min to obtain material D-B;
(D-4) The material D-B is washed by centrifugation at 1000 rcf for 5 min, resuspending in PBS buffer solution, and waiting for 30 min.

Finally, a contrast agent is obtained and denoted as D2.

### Test Example

### (1) Particle size and homogeneity of nanodroplets

The number-average particle size (Dn, unit: nm) of the nanodroplets is detected by a particle size analyzer (Malvern, Mastersizer 3000) based on the DLS principle, and the results are recorded in Table 1;

The weight-average particle size (Dw, unit: nm) of the nanodroplets is also detected by the particle size analyzer. The polydispersity coefficient=Dw/Dn is calculated, and the results are recorded in Table 1.

### (2) Stability test

The stability of the contrast agent in vivo is reflected by the half-life of the contrast agent, and the longer the half-life, the higher the stability. Particularly, the test method includes: taking a Japanese long-eared white rabbit as the experimental object, injecting the drug-loaded nanodroplets through the ear vein of the rabbit, adjusting the concentration of the drug-loaded nanodroplets to 1_{×}10¹¹/ml, and the injection dose to 0.1ml/kg; collecting blood from the long-eared white rabbits at different times, and monitoring the content of phospholipid in the blood by high performance liquid chromatography. The half-life of the contrast agent is a period corresponding to the time when the phospholipid concentration in the blood is half of the initial injection concentration.

The measured half-life results of the contrast agents from the Examples and Comparative Examples are recorded in Table 1, respectively.

### (3) Detection of ultrasound activation threshold (without optical assistance)

In vitro ultrasound activation experiment is performed, and the results are recorded in Table 1. Particularly, the test method includes: embedding drug-loaded nanodroplets into an agar model for in vitro site-specific activation experiments. Herein, the concentration of drug-loaded nanodroplets is 1×10⁹/ml, and the content of agar is 1% (w/v). The ultrasonic probe (center frequency: 7.8MHz) is placed just above the agar model, and the probe and the agar model are acoustically coupled through ultrasonic couplant, adjusting the mechanical index of the ultrasound probe, and observing the change of grayscale in the ultrasound image. Since there is a weak signal in the ultrasonic image before activation of the droplets, however, after activation there is a strong signal in the ultrasonic image due to the phase change of the droplets into microbubbles. Accordingly, the activation of the droplets may be reflected by the change of the grayscale in the ultrasonic image. In the region of interest, we set the mechanical index corresponding to the ultrasound that may induce 10% nanodroplet activation as the activation threshold of the droplets.

### (4) Detection of ultrasound activation threshold with optical assistance

In vitro site-specific ultrasound activation experiment is performed with optical assistance, and the results are recorded in Table 1. Particularly, the test method includes: embedding drug-loaded nanodroplets into an agar model for in vitro site-specific activation experiments. Herein, the concentration of drug-loaded nanodroplets is 1×10⁹/ml, and the content of agar is 1% (w/v). The ultrasonic probe (center frequency: 7.8MHz) is placed just above the agar model, and the probe and the agar model are acoustically coupled through ultrasonic couplant. The difference from the threshold of ultrasonic activation is that we irradiate the side of the agar probe with a 760nm laser, adjusting the laser energy to 1W/cm²; furthermore, adjusting the mechanical index of the ultrasound probe, and observing the change of grayscale in the ultrasound image. Since there is a weak signal in the ultrasonic image before activation of the droplets, however, after activation there is a strong signal in the ultrasonic image due to the phase change of the droplets into microbubbles. Accordingly, the activation of the droplets may be reflected by the change of the grayscale in the ultrasonic image. In the region of interest, we set the mechanical index corresponding to the ultrasound that may induce 10% nanodroplet activation as the activation threshold of the droplets with optical assistance.

### (5) Determination of ultrasound activation effect

Take the ultrasound contrast agent I1 prepared in Example 1 as an example for testing. Particularly, taking a Japanese long-eared white rabbit as the experimental object, a peripheral vein channel is established through the marginal ear vein in the left ear of the rabbit, and a three-way tube is connected at the end of the catheter, and one of the channels is used for injecting the drug-loaded ultrasound contrast agent prepared by the present invention, and one channel for injecting normal saline solution. The Japanese white rabbit is anesthetized with 3% (40 mg/kg) sodium pentobarbital. After the rabbit is fully anesthetized, the right waist is depilated to facilitate renal imaging. The concentration of the drug-loaded ultrasound contrast agent I1 prepared in Example 1 is adjusted to 1×10¹¹/ml, and then bolus injection is performed through the marginal ear vein at a dose of _{0.1}ml/kg, followed by flushing the pipeline with 1ml of normal saline solution. The B mode of the Vearsonics ultrasound imaging system is used for observation, the center frequency of the ultrasound probe is 7.8MHz, the mechanical index (MI) of the imaging ultrasound pulse before activation is 0.4, and the mechanical index of the ultrasound pulse during the activation is 1.5 (the safety threshold value of the human body is 1.9). The observed real-time ultrasound images before and after the ultrasound activation are shown in (a) and (b) of Fig. 2, respectively. It can be seen from Fig. 2 that, before activation there is dark signal in the ultrasound image since the nanodroplets are not vaporized, however, after activation there is bright signal in the ultrasound image since the nanodroplets are vaporized. Therefore, nanodroplets are capable of in vivo controlled imaging.

**Table 1:**

| | Number average particle size (nm) | Polydispersity coefficient | Half-life (h) | Ultrasound activation threshold without optical assistance | Ultrasound activation threshold with optical assistance |
|---|---|---|---|---|---|
| 11 | 270 | 0.14 | 6 | 1.4 | 0.8 |
| 12 | 250 | 0.23 | 5.5 | 1.3 | 0.8 |
| 13 | 320 | 0.20 | 6 | 1.4 | 0.8 |
| 14 | 330 | 0.24 | 5 | 1.4 | 0.9 |
| 15 | 350 | 0.26 | 5.5 | 1.3 | 0.8 |
| 16 | 260 | 0.14 | 6 | 1.4 | 1.0 |
| 17 | 220 | 0.30 | 4 | 1.7 | 1.0 |
| 18 | 220 | 0.23 | 5 | 1.4 | 0.8 |
| 19 | 260 | 0.14 | 6 | 1.4 | 1.4 |
| 110 | 270 | 0.14 | 6 | 1.4 | 1.2 |
| I11 | 380 | 0.30 | 4 | 1.3 | 0.7 |
| 112 | 270 | 0.14 | 6 | 1.4 | 0.9 |
| D1 | 420 | 0.35 | 3 | 1.8 | 1.8 |
| D2 | 520 | 0.70 | 4 | 2.3 | 2.1 |

It can be seen from Table 1, compared with the prior art, the nanodroplets of contrast agent obtained from the contrast agent composition according to the present invention have smaller particle size and higher homogeneity (lower polydispersity coefficient), higher stability (longer half-life), and lower threshold of ultrasound activation under ultrasound conditions. In addition, the contrast agent obtained by the contrast agent composition according to the present invention also has optical control properties, and the threshold of ultrasound activation may be reduced to below 1.4 with optical assistance.

The preferred embodiments of the present invention have been described above in detail; however, the present invention is not limited thereto. Within the scope of the technical concept of the present invention, a variety of simple modifications may be made to the technical solutions of the present invention, including combining various technical features in any other suitable manner. These simple modifications and combinations should also be regarded as the content disclosed in the present invention, and they all belong to the protection scope of the present invention.

## Claims

1. A film-forming agent composition for contrast agent, **characterized in that** the film-forming agent composition for contrast agent comprises a lipid, an emulsifier and a surface charge modifier;
relative to 100 parts by weight of the lipid, the content of the emulsifier is 20-50 parts by weight, and the content of the surface charge modifier is 10-35 parts by weight; and
the lipid is a carboxylated phospholipid, and the surface charge modifier is a polyelectrolyte.

2. The film-forming agent composition for contrast agent according to claim 1, **characterized in that** relative to 100 parts by weight of the lipid, the content of the emulsifier is 25-45 parts by weight, and the content of the surface charge modifier is 15-30 parts by weight.

3. The film-forming agent composition for contrast agent according to claim 1 or 2, **characterized in that** the lipid is a carboxylated phospholipid, which is any one or more selected from the group consisting of: 1,2-distearoyl-sn-glycero-3-phosphocholine, distearoylphosphatidylethanolamine, dipalmitoylphosphatidylcholine, 1,2-bis(diphenylphosphine)ethane, and distearoylphosphatidylethanolamine-polyethylene glycol.

4. The film-forming agent composition for contrast agent according to any one of claims 1-3, **characterized in that** the emulsifier is any one or more selected from the group consisting of: polyethylene glycol 4000, polyethylene glycol 40s, polyoxypropylene polyoxyethylene block polyether, polyethylene glycol 1400, and polysorbate-80.

5. The film-forming agent composition for contrast agent according to any one of claims 1-4, **characterized in that** the surface charge modifier is any one or more selected from the group consisting of: hyaluronic acid, chitosan, sodium hydroxymethyl cellulose, carbomer, sodium alginate, polyamine, and hard amine.

6. The film-forming agent composition for contrast agent according to any one of claims 1-5, **characterized in that** the film-forming agent composition for contrast agent further comprises a photosensitizer, relative to 100 parts by weight of the lipid, the content of the photosensitizer is 10-35 parts by weight, more preferably 15-30 parts by weight;
preferably, the photosensitizer is any one or more selected from: methylene blue, porphyrin, hematoporphyrin, photoporphyrin, mesoporphyrin, sodium porphyrin, gallium porphyrin, hydrophilic chlorin derivative, protoporphyrin and copper protoporphyrin.

7. The film-forming agent composition for contrast agent according to any one of claims 1-6, **characterized in that** the film-forming agent composition for contrast agent further comprises gold particles modified with amino groups on the surface, relative to the total weight of the lipid, emulsifier, surface charge modifier and photosensitizer, the content of the gold particles is 20-50 parts by weight;
preferably, the particle size of the gold particles is 1-30 nm.

8. A film-forming lipid solution for contrast agent, **characterized in that** it comprises the film-forming agent composition for contrast agent according to any one of claims 1-7, or is prepared from said film-forming agent composition for contrast agent.

9. A contrast agent, **characterized in that** it comprises a nanodroplet consisting of a shell and the content wrapped by the shell, and the shell is produced by the film-forming lipid solution for contrast agent according to claim 8.

10. The contrast agent according to claim 9, **characterized in that** the content is a biocompatible substance which comprises drug or does not comprise drug and is a gaseous state or a phase-changeable liquid;
preferably, the biocompatible substance is any one or more selected from the group consisting of: air, nitrogen, carbon dioxide, oxygen, hydrogen, nitrogen oxide, inert gas, halosilane, halosilane, haloalkane, and sulphur halide;
preferably, the content is a perfluorocarbon liquid.

11. A method for preparing a contrast agent, **characterized in that** it comprises the following steps:
(1) mixing the film-forming agent composition for contrast agent according to any one of claims 1-7 with a first organic solvent to obtain a film-forming lipid solution for contrast agent;
(2) mixing the film-forming lipid solution for contrast agent with perfluorocarbon liquid to obtain solution **A;**
(3) mixing the solution **A** and the hydration solution to produce the Ouzo effect, leaving the resulting material to stand for stratification, and separating the lipid phase to obtain material **B;**
(4) resuspending the material **B** in a buffer solution to obtain an initial nanodroplet solution;
(5) contacting the initial nanodroplet solution with gold particles modified with amino groups on the surface and an initiator.

12. The method according to claim 11, **characterized in that** in step (2), the saturability of perfluorocarbon in the solution **A** is 30-100%.

13. The method according to claim 11 or 12, **characterized in that** in step (3), the hydration solution is a mixture of glycerol, propylene glycol and phosphate in a volume ratio of 1:(0.5-3):(5-12).

14. The method according to any one of claims 11-13, **characterized in that** in step (3), the volume ratio of the solution **A** to the hydration solution is 1:(0.3-2).

15. The method according to any one of claims 11-14, **characterized in that** in step (5), the weight ratio of the amount of the initiator to that of the gold particles modified with amino groups on the surface is (0.5-4):1.
